# EUROPÄISCHE PATENTSCHRIFT

(11) **EP 1 098 935 B1**
(45) Veröffentlichungstag und Bekanntmachung des Hinweises auf die Patenterteilung: **02.01.2003**
(21) Anmeldenummer: 99931245.7
(22) Anmeldetag: 02.07.1999
(51) Int. Cl.: C08L 33/08, C08L 33/10, A61K 9/28, A61K 47/32, A61K 9/70

(54) **ÜBERZUGS- UND BINDEMITTEL FÜR ORALE ODER DERMALE ARZNEIFORMEN**
COATING AND EXCIPIENT AGENT FOR ORAL OR DERMAL DOSAGE FORMS
AGENT SERVANT D'ENROBANT ET D'EXCIPIENT POUR FORMES MEDICAMENTEUSES ORALES OU CUTANEES

(30) Priorität: 23.07.1998 DE 19833016; 23.04.1999 DE 19918435
(43) Veröffentlichungstag der Anmeldung: 16.05.2001
(73) Patentinhaber: Röhm GmbH & Co. KG, 64293 Darmstadt (DE)
(72) Erfinder: PETEREIT, Hans-Ulrich, D-64291 Darmstadt (DE); MEIER, Christian, D-64295 Darmstadt (DE); ROTH, Erna, D-64297 Darmstadt (DE)
(86) Internationale Anmeldenummer: EP9904620
(87) Internationale Veröffentlichungsnummer: WO00005307

(56) Entgegenhaltungen:
- EP-A- 0 727 205
- US-A- 4 705 695

## Beschreibung

Die Erfindung betrifft ein Überzugs- und Bindemittel für orale und dermele Arzneiformen, bestehend aus einem (Meth)acrylat-Copolymer, Weichmacher und Emulgator.

### Stand der Technik

Die Verwendung von (Meth)acrylat-Copolymeren in Arzneimittelüberzügen ist seit Jahrzehnten bekannt. (Meth)acrylat-Copolymere, die Monomerreste mit tertiären Aminogruppen enthalten, eignen sich z. B. für geschmacksisolierende Arzneimittelüberzüge, die sich im Magensaft auflösen und so eine rasche Wirkstofffreisetzung ermöglichen. In Form von organischen Lösungen lassen sich diese Polymere sehr gut verarbeiten. Organische Lösungmittel haben jedoch eine Reihe von Nachteilen, z. B. daß sie Umwelt belasten, toxisch und leicht entflammbar sind. Man ist deshalb schon seit längerem bestrebt, wo immer möglich organische Lösungen durch wäßrige Dispersionen zu ersetzen oder eine Verarbeitung ganz ohne Zusatz von Lösungsmittel zu ermöglichen (z. B. Verarbeitung in der Schmelze).

US 4 705 695 beschreibt ein Verfahren zum Überziehen von pharmazeutischen Formulierungen mit einem wäßrigen Überzugsmittel enthaltend ein wasserlösliches (Meth)acrylat-Copolymer mit tertiären Aminogruppen sowie ein wasserunlösliches, neutrales Polymer als Binder. Die Löslichkeit des (Meth)acrylat-Copolymers bestehend z. B. aus gleichen Anteilen Methylmethacrylat und Dimethylaminoethylmethacrylat, wird durch Einrühren in Pulverform mit Partikelgrößen unter 0,25 mm in Wasser unter gleichzeitiger Zugabe einer Säure bewirkt. Als Binder wird ein unlösliches Copolymer, z. B. aus Methylmethacrylat und Ethylacrylat (70 : 30), eingesetzt. Die Herstellung der Überzugslösung ist relativ aufwendig. Wegen des Gehaltes an Säure hat der Überzug einen unangenehmen Geschmack. Entsprechende Filme lösen sich sowohl in künstlichem Magensaft als auch in Wasser in weniger als zwei Minuten.

EP-B 181 515 beschreibt ein Verfahren zur Herstellung einer wäßrigen Überzugsmitteldispersion und ihre Verwendung zum Überziehen von Arzneimitteln. Hierzu wird ein in Wasser quellbares, aber nicht lösliches (Meth)acrylat-Copolymer enthaltend quaternäre Ammoniumgruppen eingesetzt. Das Copolymer kann z. B. aus Methylmethacrylat, Ethylacrylat und 2-Trimethylammoniumethlymethacrylat-Chlorid (60 : 30 : 10) bestehen. Es wird vorzugsweise in Form feingemahlenen Pulvers im Korngrößenbereich unter 200 µm eingesetzt. Korngrößen unter 20 bis 50 µm sind verwendbar, aber wegen der Neigung zum Stauben weniger zweckmäßig. Das Pulver löst sich nach längerem Rühren in Wasser bei erhöhter Temperatur auf. Die Dispergierung wird durch den Zusatz von Weichmachern gefördert. Der Zusatz von Emulgatoren wird als entbehrlich bezeichnet.

EP-A 0 727 205 beschreibt thermoplastisch verarbeitbare Überzugs- und Bindemittel für Arzneiformen. Darin ist u.a auch die lösungmittelsfreie Verarbeitung von (Meth)acrylat-Copolymeren, die Monomerreste mit tertiären Aminogruppen enthalten, mittels Zusatz von unverträglichen Fließmitteln, wie Glycerolmonostearat, zu schmelzbaren Arzneimittelformulierungen beschrieben.

### Aufgabe und Lösung

Im Gegensatz zu (Meth)acrylat-Copolymeren, die Monomerreste mit quaternären Aminogruppen aufweisen, war es bisher nicht möglich (Meth)acrylat-Copolymere, die Monomerreste mit tertiären Aminogruppen enthalten, ohne den Zusatz von Saüren in stabile wäßrige Lösungen bzw. Dispersionen zu überführen. Zudem sind meist weitere Zusätze wie neutrale (Meth)acrylat-Copolymere notwendig, um zu überhaupt sprühfähigen Formulierungen zu gelangen.

Überzüge gemäß der eingangs zitierten US 4 705 695 haben weiterhin den Nachteil, daß sie aufwendig herzustellen sind, einen bitteren Geschmack aufweisen und nur relativ aufwendig herzustellen sind. Da sich solche Formulierungen bereits in reinem Wasser schnell auflösen, sind sie als geschmacksisolierende Überzüge ungeeignet.

Es wurde als eine Aufgabe der vorliegenden Erfindung gesehen, eine Formulierung bzw. ein Verfahren zur Herstellung eines Überzugs- und Bindemittels für Arzneiformen, enthaltend (Meth)acrylat-Copolymere, die Monomerreste mit tertiären Aminogruppen, bereitzustellen, das eine einfache trockene oder wäßrige Weiterverarbeitung zuläßt. Die Formulierungen sollten sich dabei insbesondere zur Herstellung geschmacksisolierender Überzüge eignen und deshalb in Wasser eine geringere Löslichkeit als in künstlichem Magensaft aurweisen. Die wäßrigen Formulierungen sollten gut verarbeitbar sein, insbesondere beim Sprühauftrag. Weiterhin sollten sich die Formulierungen auch zur trockenen Verarbeitung eignen, um so eine weitere Alternative zu den zu schmelzbaren Arzneimittelformulierungen gemäß EP-A 0 727 205 bereitzustellen.

Überraschenderweise wurde gefunden, daß die Aufgabe gelöst wird durch ein Verfahren zur Herstellung eines Überzugs- und Bindemittels für orale oder dermale Arzneiformen bestehend aus
(a) 35 - 98 Gew.-% eines Copolymers, bestehend aus radikalisch polymerisierten C1- bis C4-Estern der Acryl- oder Methacrylsäure und weiteren (Meth)acrylat-Monomeren die funktionelle tertiäre Ammoniumgruppen aufweisen und
(b) 1 - 50 Gew.-% eines Weichmachers sowie
(c) 1 - 15 Gew.-%, eines Emulgators mit einem HLB-Wert von mindestens 14
wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt werden und das Überzugs- und Bindemittel durch Schmelzen, Gießen, Ausstreichen oder Aufsprühen hergestellt wird,
dadurch gekennzeichnet, daß
das Copolymer (a) in Pulverform mit einer mittleren Teilchengröße von 1 - 40 µm eingebracht wird.

Die überraschend gute Verarbeitbarkeit der Formulierung beruht auf der Bereitstellung des Copolymeren (a) in Pulverform mit extrem geringer Korngröße und war nicht vorhersehbar. Das Copolymer (a) in dieser Pulverform ist neu und wird ebenfalls beansprucht. Weiterhin liegt der Erfindung die Erkenntnis zugrunde, daß die Komponenten (a), (b) und (c ) in definierten Verhältnissen vorliegen müssen, um die gestellte Aufgabe zu lösen. Es wird angenommen daß die vorteilhaften Effekte auf gegenseitigen Wechselwirkungen der Komponenten miteinander während des Herstellungsverfahrens bewirkt werden. Die ebenfalls erfindungsgemäßen Überzugs- und Bindemittel werden somit durch das Herstellungsverfahren charakterisiert.

Da die vorteilhaften Effekte auch ohne Einsatz von Wasser oder Lösungsmittel erreicht werden, können die Komponenten auch in trockener Form verarbeitet werden. Es wird vermutet, daß die Bestandteile (a) und (b) und (c) dabei durch die Einwirkung von Wärme in vorteilhafter Weise miteinander aggregieren.

Die aus dem Pulver herstellbaren Schichten bzw. Überzüge sind in Wasser unlöslich oder schwerlöslich. Die verfilmte Schicht bleibt im neutralen Milieu des Mundes undurchlässig, in künstlichem Magensaft löst sie sich jedoch schnell auf und setzt den umhüllten oder eingebetteten Wirkstoff in gewünschter Weise sehr rasch frei.

### Ausführung der Erfindung

### Komponente (a)

Die Copolymere (a) bestehen im wesentlichen oder ganz aus radikalisch polymerisierten C1- bis C4-Estern der Acryl- oder Methacrylsäure und weiteren (Meth)acrylat-Monomeren die funktionellen tertiäre Ammoniumgruppen ausweisen.

Geeignete Monomere mit funktionellen tertiäre Ammoniumgruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat Besonders bevorzugt ist Dimethylaminoethylmethacrylat.

Der Gehalt der Monomere mit tertiären Ammoniumgruppen im Copolymeren kann vorteilhafterweise zwischen 30 und 70 Gew.-%, bevorzugt zwischen 40 und 60 Gew.-% liegen. Der Anteile der C1- bis C4-Estern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat, Butylacrylat.

Ein der Komponente (a) entsprechendes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein. Der Anteil der Komponente (a) an der Formulierung beträgt 35 - 98, bevorzugt 60 - 90 Gew.-%.

Die Copolymere (a) werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

Die mittlere Teilchengröße der Pulver kann wie folgt bestimmt werden:
- Durch Luftstrahlsiebung zur einfachen Aufteilung des Mahlproduktes in wenige Fraktionen. Diese Methode ist in diesem Meßbereich etwas ungenauer als die Alternativen. Mindestens 70, bevorzugt 90 % der Teilchen bezogen auf die Masse (Masseverteilung) sollen jedoch in dem erfindungsgemäßen Größenbereich von 1 - 40 µm liegen.
- Eine gut geeignete Meßmethode ist die Laserbeugung zur Bestimmung der Korngrößenverteilung. Handelsübliche Geräte erlauben die Messung in Luft (Fa. Malvern S3.01 Partikelsizer) oder bevorzugt in flüssigen Medien (Fa. LOT, Galai CIS 1). Voraussetzung für die Messung in Flüssigkeiten ist, das sich das Polymer darin nicht löst oder die Teilchen auf eine andere Weise während der Messung verändern. Ein geeignetes Medium ist z. B. eine stark verdünnte (ca. 0,02%ige) wäßrige Polysorbat 80 Lösung. Der mittlere Teilchendurchmesser muß im Bereich zwischen 1 und 40, bevorzugt zwischen 5 und 35, insbesondere zwischen 10 und 20 µm liegen.

### Komponente (b)

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glassübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wieCitrate, Phthalate, Sebacate oder Rizinusöl. Bevorzugt werden Zitronensäure- und Sebacinsäureester verwendet.

Die Zugabe der Weichmacher (b) zur Formulierung kann in bekannter Weise, direkt, in wäßriger Lösung oder nach thermische Vorbehandlung der Mischung vorgenommen werden. Auch können Mischungen von Weichmachern eingesetzt werden.

Der Anteil der Komponente (b) an der Formulierung beträgt 1 - 50, bevorzugt 5 - 30 Gew.-%.

### Komponente (c)

Emulgatoren oder Tenside sind grenzflächenaktive Substanzen mit lyobipolarem Charakter, d.h. in ihrem Molekül müssen unpolare, lipophile und polare, hydrophile Zentren vorliegen (P.H. List, Arzneiformenlehre, Wissenschaftliche Verlagsgesellschaft mbH Stuttgart, 1982, Kap. 6.2.). Je nach molekularem Aufbau unterscheidet man zwischen ionogenen und nichtionogen Emulgatoren.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 14.

Unter Emulgatoren (c) mit einem HLB-Wert über 14 werden erfindungsgemäß hydrophile, nicht ionische Emulgatoren mit HLB - Bereich von mindestens 14 sowie ebenfalls hydrophile, anionische Emulgatoren und deren Salze, die einen rechnerischen HLB-Wert über 14 aufweisen, verstanden. Emulgatoren mit HLB-Werten von weniger als 14, wie z. B. Glycerolmonostearat können zwar zusätzlich ebenfalls enthalten sein, ersetzen jedoch die Emulgatoren (c) mit HLB-Werten von mindestens 14 nicht.

Geeignete Emulgatoren (c) sind z. B. Natriumlaurylsulfat und Natriumcetylstearylsulfat, Saccharosestearat und Polysorbat 80. Die Emulatoren (c) sind in Mengen von 1 - 15, bevorzugt 5 - 10 Gew.-% enthalten. Möglich ist natürlich auch der Einsatz von Emulgatormischungen.

Die Zugabe der Emulgatoren (c) zur Formulierung kann in bekannter Weise, direkt, in wäßriger Lösung oder nach thermischer Vorbehandlung der Mischung vorgenommen werden.

Die Emulgatoren können je nach Typ und zugesetzter Menge die Funktionalität der Polymerschicht beeinflussen

### Weitere Zuschlagstoffe

Der erfindungsgemäßen Formulierung werden in der Regel bei der Verarbeitung zu Überzugs- und Bindemittels übliche Zuschlagstoffe hinzugefügt.

Einsatzmengen und Verwendung der üblichen Zuschlagstoffe in Arzmeimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.
- Trennmittel:
   Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca-Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis *100* Gew.-% bezogen auf das Copolymer (a).
   In einer besonders vorteilhaften Ausführungsform erfolgt der Zusatz des Trennmittels in konzentrierter Form als Endschicht. Der Auftrag erfolgt als Pulver oder aus wäßriger Suspension mit 5 - 30 % Feststoffgehalt durch Sprühen. Die notwendige Menge ist niedriger als bei der Einarbeitung in die Polymerschicht und beträgt 0,1 - 2 % bezogen auf das Gewicht der Arzneiform.
- Pigmente:
   Der Zusatz erfolgt nur selten in Form des löslichen Farbstoffs. In der Regel dispergiert man Aluminium- oder Eisenoxidpigmente. Titandioxid dient als Weißpigment . Übliche Einsatzmengen für Pigmente in den erfindungsgemäßen Überzugs- und Bindemitteln zwischen 20 und 60 Gew.-%, bezogen auf die Polymermischung. Wegen des hohen Pigmentbindevermögens können jedoch auch Mengen bis zu 100 Gew.-% verarbeitet werden.
   In einer besonders vorteilhaften Ausführungsform erfolgt der Einsatz direkt in konzentrierter Form als Endschicht. Der Auftrag erfolgt als Pulver oder aus wäßriger Suspension mit 5 - 30 % Feststoffgehalt durch Sprühen. Die notwendige Menge ist niedriger als bei der Einarbeitung in die Polymerschicht und beträgt 0,1 - 2 % bezogen auf das Gewicht der Arzneiform.
   Grundsätzlich müssen natürlich alle eingesetzten Substanzen toxikologisch unbedenklich und in Arzneimitteln ohne Risiko für Patienten zu verwenden sein.

### Das Herstellungsverfahren

Die Komponenten (a), (b) und (c) werden mit oder ohne Zusatz von Wasser und gegebenenfalls unter Zusatz eines pharmazeutischen Wirkstoffs und der weiteren üblichen Zuschlagstoffe miteinander vermengt werden und das Überzugs- und Bindemittel durch Schmelzen, Gießen, Ausstreichen oder Aufsprühen hergestellt. Dabei ist die Verfilmung des Überzugs- und Bindemittels ist Voraussetzung für den funktionellen Effekt in Arzneiformen.

Die Verfilmung erfolgt, unabhängig von dem Auftragsverfahren durch Zuführung von Energie. Dies kann über Konvektion (Wärme), Strahlung (Infrarot oder Mikrowellen) oder Leitung erfolgen. Für den Auftrag als Suspensionsmittel eingesetztes Wasser verdampft dabei, gegebenenfalls kann auch ein Vakuum angewendet werden, um das Verdampfen zu beschleunigen. Die für die Verfilmung notwendige Temperatur hängt von der Kombination der eingesetzten Komponenten ab.

### Verwendung der erfindungsgemäßen Formulierung zur Herstellung von Bindemitteln:

Die Verwendung als Bindemittel erfolgt z. B. durch Aufsprühen der wäßrigen Polymersuspension auf wirkstofffreie Kerne (Nonpareilles) bei gleichzeitiger Zugabe von pulverförmigem Wirkstoffen oder deren Mischungen.
Eine weitere Ausführungsform ist das Aufsprühen der wäßrigen Polymersuspension gemeinsam mit darin gelösten oder suspendierten Wirkstoffen.

### Venrwendung der erfindungsgemäßen Formulierung zur Herstellung von Überzugsmittel:

Träger für die Überzüge sind Kapseln, Tabletten, Granulate, Pellets, Kristalle von regelmäßiger oder unregelmäßiger Form. Die Größe von Granulaten, Pellets oder Kristallen liegt zwischen 0,01 und 2,5 mm, die von Tabletten zwischen 2,5 und 30,0 mm. Kapsel bestehen aus Gelatine, Stärke oder Cellulosederivaten.

Sie enthalten in der Regel die biologisch aktive Substanz (Wirkstoff) bis zu 95 % sowie weitere pharmazeutische Hilfsstoffe bis zu 99,9 Gew.-% Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Von besonderer Bedeutung ist die Zerfallszeit der Kerne, die die Freigabe des Wirkstoffs beeinflußt. Man strebt heute kurze Zerfallszeiten von unter 5, bzw. unter 10 min im Zerfallstest nach Ph. Eur. an. Längere Zerfallszeiten sind deswegen problematisch, weil zusätzliche Überzüge die Freigabe des Wirkstoffs weiter verzögern und den therapeutischen Effekt in Frage stellen können. Als Grenzwert wird heute eine Zerfallszeit von 30 min angesehen. Man testet in Wasser und künstlichem Magensaft (0,1 N HCl). Für die Funktion von Polymeren mit tertiären Aminogruppen ist der pH - Einfluß auf den Zerfall der Kerne oder die Wirkstoffabgabe wichtig. Ausreichende Funktionalität ist gegeben, wenn die Zerfallszeit in Wasser mindestens doppel so lang ist wie in künstlichem Magensaft.

Die eingesetzten Kerne sind homogen oder haben einen schichtartigen Aufbau. Sind Gravuren in die Oberflächen eingelassen, sollen diese durch Überzüge möglichst überdeckt aber wenig ausgefüllt werden. Die erfindungsgemäß eingesetzte Schichtdicke des Polymerpulvers variiert stark und hängt von dem Verarbeitungsverfahren oder der Menge an Zuschlagstoffen ab. Sie liegt zwischen 1 und 100 µm, bevorzugt zwischen 10 und 50 µm. Auf üblichen Tabletten entspricht das einem Polymerauftrag von 0,5 bis 5 Gew.-%.
Überzogen Mikropartikel können gemäß K. Lehmann et al., Drugs made in Germany 37, 2, 53-60 (1994) und T.E. Beckert et al, International Journal of Pharmaceutics 143,(1996), 13-23 zu zerfallenden Tabletten verpreßt werden, ohne signifikanten Einfluß auf die Funktion des Polymers.

Die Funktion der verfilmten Polymerschicht in der endgültigen Arzneiform kann vielfältig sein:
- Schutz vor schädlichen Umwelteinflüssen durch Feuchte, Gase, Licht usw.
- Geruchs- oder Geschmacksisolierung,
- Kennzeichnung durch Farbe
- Mechanische Stabilisierung
- Isolierung unverträglicher Inhaltsstoffe
- Vermeidung von Haftung an den Schleimhäuten.
- Zeitlich verzögerte Wirkstoffabgabe
- pH - gesteuerte Wirkstoffabgabe

Vorteilhaft ist die niedrige Viskosität der Polymermischung in wäßriger Dispersion auch bei hohen Feststoffanteilen bis zu 30 %, da Gravuren auf der Oberfläche von Tabletten detailliert nachgebildet werden.
Besonders vorteilhaft ist die gute Schutz- und Isolierwirkung der erfindungsgemäßen Polymermischung bei gleichzeitig geringem Einfluß auf den Tablettenzerfall. Schon bei geringen Polymeraufträgen von 1 Gew.-% schon eine Geschmacksisolierung von mehr als 30 sec. erreicht. Dickere Überzüge mit einem Copolymer aus Methylmethacrylat, Butylmethacrylat, und Dimethylaminoethylmethacrylat in Verhältnis 25 : 25 : 50 (EUDRAGIT® E 100) verbessern die Geschmacksabdeckung, ohne jedoch die Zerfallszeit in 0,1N HCI zu verlängern. Ebenso vorteilhaft ist die zuverlässige Abdeckung gefärbter Kerne durch Überzüge mit hohem Pigmentanteil. Eine besondere Ausführungsform ist die Einbettung eines zweiten Wirkstoffs in den Überzug auf einen wirkstoffhaltigen Kern.

### Auftrag auf der erfindungsgemäßen Formulierung zur Herstellung auf Träger

Die erfindungsgemäße Formulierung kann in Pulverform, als Schmelze oder in wäßriger Suspension durch Gießen, Ausstreichen oder mittels Sprühauftrag angewendet werden. Wasser dient dabei hauptsächlich als Vehikel, um dünne Umhüllungen gleichmäßig auf sphärische Kerne z. B. durch Sprühen aufzutragen. Für Beschichtungen werden außerdem Streichverfahren eingesetzt. Das eingesetzte Verfahren richtet sich hauptsächlich nach dem gewählten Träger. Trockene Pulver werden durch Ausstreichen oder Bestäuben aufgetragen, ggf. auch unter Einsatz elektrostatische Kräfte. Für die Ausführung ist dabei entscheidend; daß gleichmäßige, geschlossene Schichten entstehen.

Auftragsverfahren gemäß dem Stand der Technik s. z. B. Bauer, Lehmann, Osterwald, Rothgang, "Überzogene Arzneiformen" Wissenschaftliche Verlagsgesellschaft mbH Stuttgart, Kap 7, S.165 - 196
Für die Applikation relevante Eigenschaften, geforderte Tests und Spezifikationen sind in Arzneibüchern aufgelistet.
Details sind den gängigen Lehrbüchern zu entnehmen, z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626-642.
- Gennaro, A.,R. (Editor), Remington's Pharmaceutical Sciences. Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Die erfindungsgemäßen Überzugs- und Bindemittel können als Bestandteil eines transdermalen Therapiesystems verwendet werden. Im typischen Fall handelt es sich hierbei um ein Pflaster, das einen pharmazeutischen Wirkstoff enthält, der nach der Freigabe lokal wirkt oder durch die Haut in die Blutbahn aufgenommen, im Körper verteilt und dort systemisch wirkt.

Dermale und Transdermale Therapiesysteme haben oft einen mehrschichtigen Aufbau und werden gemäß dem strukturellen Aufbau unterschieden in:
- Reservoir Systeme
- Matrix Systeme
- Drug-in-Adhesive Systeme
- Multi-Laminate Systeme

Der Arzneistoff ist in eine oder mehreren Schichten dieser Systeme eingebettet und wird nach dem Fixieren auf der Haut kontrolliert abgegeben um die gewünschte Wirkung zu entfalten.

Folgende Wirkstoffe (bzw. deren Saize) sind bereits in Handelspräparaten enthalten oder befinden sich in Entwicklungen:
Nicotin, Glyceroltrinitrat, Scopolamin, Clonidin, Fentanyl, Östradiol, Testosteron, Oxibutynin, Diclophenac, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, diverse Peptide, Eptazocine, Ethinylöstradiol, Methotrexat, Naloxon, und Tretinion

In einer besonderen Ausführungsform beschichtet man antiadhäsive Träger und verfilmt das erfindungsgemäße Überzugs- und Bindemittel. Anschließend trennt man den erhaltenen Film von der Unterlage und setzt ihn in freier Form zum Laminieren, Kalandrieren oder Umhüllen ein. Die notwendige Haftung erreicht man durch Erwärmen, Verkleben. Dabei kann zur Stabilisierung zusätzlich Druck eingesetzt werden.

### Biologisch aktive Substanzen:

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder.
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.
Erfindungsgemäß können alle Wirkstoffe eingesetzt werden, die gewünschte therapeutische Wirkung im Sinne der obigen Definition erfüllen und eine ausreichende thermische Stabilität besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.
Bevorzugte Wirkstoffe für verzögerte Wirkstoffabgabe sind:
Nifedipin, Diltiazem, Theophyllin, Diclofenac Na, Ketoprofen, lbuprofen,
Indometazin, Ambroxol, Terbutalin, Vincamin, Propranolol, Pentoxyphyllin,
Kodein, Morphin, Etilefrin, Carbamazepin bzw. deren therapeutische eingesetzte Salze.

### Applikationsformen:

Grundsätzlich können die beschriebenen Arzneiformen direkt durch orale Applikation zur Anwendung kommen. Die erfindungsgemäß hergestellten Granulate, Pellets, oder Partikeln können in Gelatinekapseln, Beutel (Sachets) oder in geeignete Mehrdosenbehälter mit Dosiereinrichtung abgefüllt werden. Die Einnahme erfolgt in fester Form oder suspendiert in Flüssigkeiten.
Durch Verpressen erhält man aus, ggf. nach Zumischung weiterer Hilfsstoffe, Tabletten die nach der Einnahme zerfallen und die meist überzogenen Untereinheiten freisetzen. Denkbar ist ebenso die Einbettung von Agglomeraten in Polyethylenglykol oder Lipide zur Herstellung von Suppositorien oder vaginalen Arzneiformen. Überzogene Tabletten werden in Blister oder Mehrdosenbehälter verpackt und vom Patienten direkt vor der Einnahme entnommen.

### BEISPIELE

Rezepturen und galenische Daten der in den Beispielen verwendeten Tabletten werden in der folgenden Tabelle beschrieben:

| Inhaltsstoffe | Placebo-Tabletten | Chinidinsulfat-Tabletten | Methylenblau Tabletten |
|---|---|---|---|
| Cellactose® | - - | 92,5% | |
| Avicel® PH 102 | 30,0% | 5,0 % | 30,0% |
| Mg-Stearat | 0,3% | 0,5 % | 0,3% |
| Chinidinsulfat | - - | 2,0 % | |
| Laktose D 20 | 61,2% | - - | 59,2% |
| Aerosil 200 | 0,5% | - - | 0,5% |
| Talkum | 3,0% | - - | 3,0% |
| Amijel | 5,0% | - - | 5,0% |
| Methylenblau | | | 2,0% |
| Aussehen | weiß | weiß | weiß-blau |
| Durchmesser | 8,0 mm | 10,0 mm | 7,0mm |
| Höhe | 3,95 mm | 3,91 mm | 4,06 |
| Gewicht | 191 - 210 mg | 398 - 312 mg | 142mg |
| Härte | 93 - 102 N | 113 - 133 N | >50N |
| Zerfall in demineralisiertem Wasser | 15-40 sec | 13 - 20 min | 15-60sec |
| Zerfall in 0,1 N HCI | 15-42 sec | 14 - 20 min | 10 -37 sec. |

Alle eingesetzten Hilfsstoffe haben pharmazeutische Qualität.
Die Herstellung der in einigen Beispielen eingesetzten wäßrigen Suspension von Glycerolmonostearat (GMS) und erfolgt durch Suspendieren von GMS in einer entsprechenden Menge Wasser, Erhitzen auf ca. 60 °C und Abkühlen des Ansatzes auf Raumtemperatur unter Homogenisierung mit einem Intensivmischer (z. B. Ultra Turrax).

Die Pulver aus dem Copolymer (a) aus Methylmethacrylat, Butylmethacrylat, und Dimethylaminoethylmethacrylat in Verhältnis 25 : 25 : 50. (EUDRAGIT® E100) wurde hergestellt durch Vermahlung vom extrudierten Granulaten auf verschiedenen Luftstrahlmühlen (erfindungsgemäße Teilchengrößen) oder einer Stiftmühle (nicht erfindungsgemäße Teilchengröße)

Die Bestimmung der Teilchengröße erfolgte in einem Particlesizer S3.01 (Fa. Malvern Instruments) oder in einem Galai CIS 1 (Fa. LOT).

Die Beispiele 1 bis 3 beschreiben den direkten Auftrag des erfindungsgemäßen Überzugs- und Bindemittels als Pulver oder Paste.

In Beispielen 4 bis 17 werden Rezeptur- und Ausführungsvarianten aus wäßriger Suspension beschrieben.

Die Beispiele 18 bis 20 beschreiben nicht erfindungsgemäße Ausführungsformen (Vergleichsbeipiele)

### 1. Herstellung einer Isolierschicht mit einem hydrophilen Weichmacher und einem nichtionischen Emulgator

3,5 g eines Copolymers aus Methylmethacrylat, Butylmethacrylat, und Dimethylaminoethylmethacrylat in Verhältnis 25 : 25 : 50. (EUDRAGIT® E 100) wird im Mörser mit 5g Polyethylenglykol 12000 sowie 1,5 g Sacharosestearat vermischt. Das erhaltene Pulver wird gleichmäßig auf Teflonfolie ausgestrichen und in einem Trockenschrank bei 100°C über ca. 15 Stunden verfilmt. Es bildet sich ein zusammenhängender klarer Film, der sich in demineralisiertem Wasser nicht löst. Er kann z. B. im Transferverfahren auf flächige Träger übertragen werden oder als freier Film zum Umhüllen kubischer Kerne dienen. Dabei können einzelne Schichte miteinander verklebt oder verschweißt werden.

### 2. Hersteflung einer Isolierschicht mit einem hydrophilen Weichmacher und einem nichtionischen Emulgator

In einem Mörser werden 10,0 g ) eines Copolymers aus Methylmethacrylat, Butylmethacrylat, und Dimethylaminoethylmethacrylat in Verhältnis 25 : 25 : 50 (mittlere Teilchengröße 10 µm), 5 g TEC, und 1,5g Polysorbat 80 vermischt. Diese milchig weiße, hochviskose Paste wird auf einer teflonisierten Glasplatte ausgestrichen und wie in Beispiel 1 verfilmt. Es bildet sich ein klarer, klebriger Film, der in demineralisiertem Wasser unlöslich ist und wie in Beispiel 1 eingesetzt werden kann.

### 3. Herstellung einer Isolierschicht mit einem lipophilen Weichmacher und einem nichtionischen Emulgator

In einem Mörser werden 5 g Dibutylsebakat, 1,5 g Sacharosestearat und 5 g eines Copolymers aus Methylmethacrylat, Butylmethacrylat, und Dimethylaminoethylmethacrylat in Verhältnis 25 : 25 : 50 (mittlere Teilchengröße 10 µm) vermischt. Diese milchig weiße, hochviskose Paste wird auf einer teflonisierten Glasplatte ausgestrichen und wie in Beispiel 1 verfilmt. Es bildet sich ein klarer, klebriger Film, der in demineralisiertem Wasser unlöslich ist und wie in Beispiel 1 eingesetzt werden kann.

### 4. Farbloser Isolierüberzug mit einem nichtionischen Emuigator und einem hydrophilen Weichmacher

In ein Becherglas werden 274 g Wasser, 18 g einer 33,3%igen Polysorbat 80 Lösung und 9 g Triethylcitrat mit einem Flügelrührer gemischt. Anschließend wird lamgsam unter Rühren 60 g eines Copolymers aus Methylmethacrylat, Butylmethacrylat, und Dimethylaminoethylmethacrylat in Verhältnis 25 : 25 : 50(mittlerer Teilchendurchmesser 15,3 µm) zugegeben und 90 min weitergerührt.

Nun wird zu der Mischung 20 g einer 6%igen Glycerolmonostearat-Suspension dazugegeben und mit einem Ultra Turrax nochmals 10 min mit einer Drehzahl von 3500/min homogenisiert.

Die erhaltene Sprühsuspension wird in einem Dragierkessel (25 cm ⌀, Drehzahl ca 40/min) auf 1500 g Placebo-Tabletten mit einer Sprühpistole ( Sprühdruck 0,8 bar) aufgesprüht. Durch Einleiten von vorgewärmter Luft wird die Produkttemperatur auf 25 - 40°C gehalten und eingesetzes Wasser verdampft. Der Polymerauftrag liegt bei 4 mg/cm². Die Sprühzeit beträgt 60 min. Zum Schluß wird mit 3 g Magnesiumstearat abgestreut und über Nacht bei RT getrocknet. Die Tabletten weisen eine glatte glänzende Oberfläche auf und zeigen im Zerfallstest nach Ph. Eur. in demineralisiertem Wasser und künstlichem Magensaft folgende Werte:

| | Polymerauftrag | |
|---|---|---|
| | 4 mg/cm² | unüberzogen |
| Wasser demin. | 15->20 min | 0,2-0,7 min |
| 0,1 N HCl | 0,3-1 min | 0,2-0,7 min |

### 5. Farbloser Isolierüberzug mit einem ionischen Emulgator und einem lipohilen Weichmacher

In einem Becherglas werden 30,0 g eines Copolymers aus Methylmethacrylat, Butylmethacrylat, und Dimethylaminoethylmethacrylat in Verhältnis 25 : 25 : 50(mittlerer Teilchendurchmesser 13,3 µm), und 3,0 g Natriumlaurylsulfat, 150 g demineralisiertes Wasser gegeben und 6 g Dibutylsebakat vermischt und 1,5 Stunden gerührt. Dann wird zu der Suspension 20 g Talkum und 80 g demineralisiertes Wasser dazugegeben und weitere 10 min gerührt.
Die erhaltene Sprühsuspension wird wie in Beispiel 4. beschrieben auf Placebotabletten aufgetragen. Der Gesamtpolymerauftrag ist 2 mg/cm². Die Sprühzeit beträgt 71 min. Anschließend wird bei Raumtemperatur über Nacht getrocknet. Die Tabletten zeigen einen glänzenden gleichmäßigen Überzug und zeigen im Zerfallstest nach Ph. Eur. in demineralisiertem Wasser und künstlichem Magensaft folgende Werte:

| | Polymerauftrag | | |
|---|---|---|---|
| Medium | 1 mg/cm² | 2 mg/cm² | unüberzogen |
| Demineralisiertes Wasser | 1,5 - 2 min | 1,5-2,3 min | 0,2-0,7 min |
| 0,1 N HCl | 1 min | 1-1,3 min | 0,2-0,7 min |

### 6. Farbloser Isolierüberzug mit einem ionischen Emulgator und einem lipohilen Weichmacher

In einen Glasreaktor werden 123,0 g eines Copolymers aus Methylmethacrylat, Butylmethacrylat, und Dimethylaminoethylmethacrylat im Verhältnis 25 : 25 : 50 mit 4,5 g Natriumlaurylsulfat und 600 g demineralisiertes Wasser gegeben und mit der Disolverscheibe mit einer Drehzahl von 600 /min unter 720 mbar Vakuum gerührt, auf 45°C aufgeheizt und bei dieser Temperatur 1,5 Stunden gerührt. Anschließend gibt man 18,0 g Dibutylsebakat langsam hinzu und kühlt unter Rühren innerhalb von 60 min auf 20°C ab. Nun wird nochmals 4,5 g Natriumlaurylsulfat zugegeben und bis insgesamt 5 Stunden weitergerührt. 183 g dieser Dispersion werden in einem Becherglas mit 30 g Talkum und 40 g demineralisiertem Wasser gemischt und ca. 20 min gerührt.
Die erhaltene Sprühsuspension wird wie in Beispiel 4. beschrieben auf Placebotabletten aufgetragen. Der Gesamtpolymerauftrag ist 2 mg/cm². Die Sprühzeit beträgt 46 min. Anschließend wird über Nacht bei Raumtemperatur getrocknet. Die Tabletten weisen einen gleichmäßigen, glänzenden Überzug auf, und zeigen im Zerfallstest nach Ph. Eur. in demineralisiertem Wasser und künstlichem Magensaft folgende Werte:

| Medium | Polymerauftrag | | |
|---|---|---|---|
| | 11 mg/cm² | 2 mg/cm² | unüberzogen |
| Demin. Wasser | 3,3-4,5 min | 4,5-6,5 min | 0,2-0,7 min |
| 0,1 N HCl | 0,7-1,2 min | 0,8-1,3 min | 0,2-0,7 min |

### 7. Farbloser Isolierüberzug mit einem ionische Emulgator und einem lipohilen Weichmacher

In einen Glasreaktor werden 131,8 g einer extrudierten und gemahlenen Mischung (mittlere Teilchengröße 4,5 µm) eines Copolymers aus Methylmethacrylat, Butylmethacrylat, und Dimethylaminoethylmethacrylat in Verhältnis 25 : 25 : 50 und Glycerolmonostearat (70/30), 7,3 g Natriumlaurylsulfat sowie 10,94 g Dibutylsebakat in 600 g demineralisiertem Wasser mit der Disolverscheibe über 5 Std. bei einer Drehzahl von 400 /min unter 720 mbar Vakuum bei 30 °C dispergiert.
Die erhaltene Sprühsuspension wird wie in Beispiel 4. beschrieben auf Placebotabletten aufgetragen. Der Polymerauftrag ist 2mg /cm². Die Sprühzeit beträgt 84 min. Die Tabletten werden bei Raumtemperatur über Nacht getrocknet. Sie weisen einen gleichmäßigen, glänzenden Überzug auf und zeigen im Zerfallstest nach Ph. Eur. in demineralisiertem Wasser und künstlichem Magensaft folgende Werte:

| Medium | Polymerauftrag | | |
|---|---|---|---|
| | 1 mg/cm² | 2mg/cm² | unüberzogen |
| Demin. Wasser | 1,5-2,1 min | 2,1-3,0 min | 0,2-0,7 min |
| 0,1 N HCI | 0,7-1,0 min | 0,8-1,3 min | 0,2-0,7 min |

### 8. Farbloser Isolierüberzug mit einem ionischen Emulgator und einem lipohilen Weichmacher mit Endüberzug

In einem Becherglas werden 6 g Natrium-Cetylstearylsulfat (Lanette E) in 288 g demineralisiertem Wasser bei 70°C gelöst. Nun werden langsam 60 g eines Copolymers aus Methylmethacrylat, Butylmethacrylat, und Dimethylaminoethylmethacrylat in Verhältnis 25 : 25 : 50 (mittlerer Teilchendurchmesser 15,3 µm) und 6 g Dibuthylsebakat zugegeben. Nun wird eine Stunde bei dieser Temperatur auf dem heizbaren Magnetrührer weitergerührt, auf ca. 50°C abgekühlt, weitere 3 g Dibuthylsebacat dazugegeben und auf Raumtemperatur abgekühlt. In dem Ansatz werden 30 g Talkum dispergiert.

Die erhaltene Sprühsuspension wird wie in Beispiel 4. beschrieben auf Placebotabletten aufgetragen. Der Polymerauftrag ist 4 mg /cm². Die Sprühzeit beträgt 62 min. Anschließend wird noch innerhalb von 5 min 40 g einer wäßrigen, 18,7%igen Talkumsuspension aufgesprüht. Die Tabletten 2,5 Std. bei 40°C im Trockenschrank getrocknet. Die Tabletten weisen einen gleichmäßigen, glänzenden Überzug auf.
Sie zeigen im Zerfallstest nach Ph. Eur. in demineralisiertem Wasser und künstlichem Magensaft folgende Werte:

| Medium | Polymerauftrag | | |
|---|---|---|---|
| | 2 mg/cm² | 4 mg/cm² | unüberzogen |
| Demin. Wasser | 2-2,5 min | 3,7-6 min | 1-13 sec |
| 0,1 N HCl | 1-1,3min | 1-1,5 min | 1-13 sec |

### 9. Farbiger Isolierüberzug mit einem ionischen Emulgator und einem lipophilen Weichmacher

In ein Becherglas werden 370 g demineralisiertes Wasser, 4,2 g Natriumlaurylsulfat und 9 g Dibutylsebacat 3 min gemischt und langsam unter Rühren mit 60 g eines Copolymers aus Methylmethacrylat, Butylmethacrylat, und Dimethylaminoethylmethacrylat in Verhältnis 25 : 25 : 50 (mittlerer Teilchendurchmesser 15,3 µm) versetzt und 3,5 stunden bei Raumtemperatur gerührt. Zu dieser Suspension gibt man 100 g einer 20 %igen Talkumsuspension und vermischt gleichmäßig.

Die erhaltene Sprühsuspension wird wie in Beispiel 4. beschrieben auf Placebotabletten aufgetragen. Der Polymerauftrag ist 4 mg /cm². Die Sprühzeit beträgt 95 min. Anschließend werden innerhalb von 15 min 55 g Pigment-Suspension (bestehend aus 0,2 % Polysorbat 80, 14,9 % Talkum, 2,1 % Magnesiumstearat, 6,4 % Titandioxid, 6,4 % Chinolingelb, 2,1 % Polyethylenglykol 6000 und 67,9 % Wasser). Die Tabletten werden bei Raumtemperatur über Nacht getrocknet. Sie weisen einen glatten, glänzenden Überzug auf. Sie zeigen im Zerfallstest nach Ph. Eur. in demineralisiertem Wasser und künstlichem Magensaft folgende Werte:

| Medium | Polymerauftrag | | |
|---|---|---|---|
| | 2 mg/cm² | 4 mg/cm² | unüberzogen |
| Demin. Wasser | 4,5-9,4 min | 7,4 - > 20 min | 1-13 sec |
| 0,1 N HCI | 0,5-1,0 min | 1,0-1,1 min | 1-13 sec |

### 10. Farbiger Isolierüberzug mit einem nichtionischen Emulgator und einem hydrophilen Weichmacher

In einem Becherglas werden 333,5 g demineralisiertes Wasser, 12,6 g einer 33,3%igen Polysorbat 80 Lösung und 9 g Triethylcitrat mittels eines Flügelrührers vorgemischt und langsam 60 g eines Copolymers aus Methylmethacrylat, Butylmethacrylat, und Dimethylaminoethylmethacrylat in Verhältnis 25 : 25 : 50 (mittlerer Teilchendurchmesser 15,3 µm) eingerührt und 1,5 Stunden bei Raumtemperatur gerührt. Anschließend werden 50 g einer 10%igen Talkumsuspension dazugeben und mit Ultra Turrax 10 min bei 3000/min homogenisiert und mit 2-3 Tropfen Antischaumemulsion auf dem Magnetrührer versetzt.
Die erhaltene Sprühsuspension wird wie in Beispiel 4. beschrieben auf Placebotabletten aufgetragen. Der Polymerauftrag ist 4 mg/cm². Die Sprühzeit beträgt 70 min. Anschließend werden innerhalb von 15 min 55 g einer Pigment-Suspension (gemäß Beispiel 9.) aufgesprüht. Die Hälfte der Tabletten werden bei Raumtemperatur über Nacht , die andere Hälfte 6 Std. bei 40°C im Trockenschrank getrocknet. Die Tabletten weisen einen glatten, glänzenden Überzug auf. Sie zeigen im Zerfallstest nach Ph. Eur. in demineralisiertem Wasser und künstlichem Magensaft folgende Werte:

| Medium | Polymerauftrag 4 mg/cm² | | unüberzogen |
|---|---|---|---|
| | Trocknung bei RT | Trocknung bei 40 °C | |
| Demin. Wasser | 3,2-5,5 min | 1,5-7,1 min | 1-13 sec |
| 0,1 N HCI | 0,7,0-1,0 min | 0,7-1,0 min | sec |

### 11. Farbiger Isolierüberzug mit einem nichtionischen Emulgator und einem hydrophilen Weichmacher

In einem Becherglas werden 175 g demineralisiertes Wasser, 18 g einer 33,3%igen Polysorbat 80 Lösung und 9 g Triethylcitrat bei Raumtemperatur gemischt. Dann werden langsam 60 g eines Copolymers aus Methylmethacrylat, Butylmethacrylat, und Dimethylaminoethylmethacrylat in Verhältnis 25 : 25 : 50 (mittlerer Teilchendurchmesser 15.3 µm) dazugegeben und über Nacht gerührt. Dann werden 40 g einer 3 %igen Glycerolmonostearat-Suspension dazugegeben und mit einem Ultra Turrax bei einer Drehzahl von 3000/min 10 min homogenisiert, mit Wasser auf 400 g verdünnt und mit 2 Tropfen Antischaumemulsion versetzt.

Die erhaltene Sprühsuspension wird wie in Beispiel 4. beschrieben auf Placebotabletten aufgetragen. Der Polymerauftrag beträgt 4 mg/cm². Die Sprühzeit beträgt 56 min. Nach dem Auftrag werden 25g einer 3 %igen GMS-Suspension innerhalb von 5 min aufgesprüht. Um einen farbigen Überzug zu erhalten wird nochmals 35 g einer Pigmentsuspension (bestehend aus 0,2 % Polyethylenglykol 6000, 2,4 % GMS, 2,4% Titandioxid, 2,4% Chinolingelb, und 92,6% Wasser.) innerhalb 8 min aufgetragen. Die Hälfte der Tabletten werden Raumtemperatur über Nacht, die andere Hälfte 6 Std. bei 40°C im Trockenschrank getrocknet. Sie weisen einen glatten, gleichmaäßig gefärbten Überzug auf. Sie zeigen im Zerfallstest nach Ph. Eur. in demineralisiertem Wasser und künstlichem Magensaft folgende Werte:

| Medium | Polymerauftrag | | | |
|---|---|---|---|---|
| | 3 mg/cm² Trocknung bei RT | 4 mg/cm² Trocknung bei RT | 4 mg/cm² Trocknung bei 40 °C | unüberzogen |
| demin. Wasser | 2,4-6,4 min | 15 - > 20 min | 6,3- >20 min | 1-13 sec |
| 0,1 N HCl | 0,3-0,6 min | 0,5-0,75 min | 28-45 sec. | 1-13 sec |

### 12. Pulverbeschichtung mit anschließender Verfilmung durch Infrarot Strahlung

In ein Becherglas werden 85,0 g demineralisiertes Wasser, 9 g einer 33,3%igen Polysorbat 80 Lösung, 6 g ATBC gemischt, darin 30g eines Copolymers aus Methylmethacrylat, Butylmethacrylat, und Dimethylaminoethylmethacrylat in Verhältnis 25 : 25 : 50 (mittlerer Teilchendruchmesser 15,3 µm) suspendiert und eine Stunde gerührt.

Die erhaltene Polymersuspension wird mit einer Rakel in ein 500 µm dicken Schicht auf eine Glasplatte aufgetragen und unter einer Infrarotlampe bei 80 °C über 5 min verfilmt. Es entsteht ein klarer glänzender Film, der sich nicht in Wasser löst.

### 13. Pulverbeschichtung mit anschließender Verfilmung durch Infrarot Strahlung

In einem Becherglas werden 81,5 g demineralisiertes Wasser, 9 g einer wäßrigen 33,3 %igen Polysorbat 80 Lösung, 4,5 g TEC gemischt, darin 30 g eines Copolymers aus Methylmethacrylat, Butylmethacrylat, und Dimethylaminoethylmethacrylat in Verhältnis 25 : 25 : 50 (mittlerer Teilchendurchmesser 15,3 µm) suspendiert und 1 Stunde gerührt. Die erhaltene Polymersuspension wird wie in Beispiel 12 beschichtet und verfilmt. Man erhält einen klaren Film, der sich in Wasser nicht löst.

### 14. Pulverbeschichtung mit anschließender Verfilmung durch Mikrowellen

Eine Polymersuspension gemäß Beispiel 12 wird wie dort beschrieben aufgetragen und in einem in einem Microwellen-Kombinationsgerät NE-972-973 (Firma Panasonic)) über 12 min bei 360 W verfilmt.
Es entsteht ein klarer glänzender Film, der sich nicht in Wasser löst.

### 15. Pulverbeschichtung mit anschließender Verfilmung durch Mikrowellen

Eine Polymersuspension gemäß Beispiel 13 wird wie dort beschrieben aufgetragen und wie in Beispiel 14 verfilmt. Es entsteht ein klarer, flexibler und glänzender Film, der sich in Wasser nicht löst.

### 16. Geschmacksisolierender Überzug

Der in Beispiel 4 beschriebene Versuch wird mit Chinidinsulfat Tabletten wiederholt. Eine sensorische Prüfung ergab folgende Zeiten für die Maskierung des bitteren Geschmacks:

| | Polymerauftrag | | | |
|---|---|---|---|---|
| | 1 mg /cm² | 2mg/cm² | 4mg/cm² | Unüberzogen |
| Geschmacks-isolierung | 20 sec. | 5 min | 10 min | 1 -13 sec |

### 17. Isolierüberzug gegen Feuchtigkeit

In einer Kolloidmühle MZ 50 (Fa. Fryma) werden 1540 g demineralisiertes Wasser, 400,0 g eines Copolymers aus Methylmethacrylat, Butylmethacrylat, und Dimethylaminoethylmethacrylat in Verhältnis 25 : 25 : 50 (mittlerer Teilchendurchmesser 15,3 µm), 20,0 g Natriumlaurylsulfat und 40,0 g Dibutylsebakat über 1,5 Stunden dispergiert.

In 388,0 g dieser Suspension werden 34,0 g Talkum und 433,0 g demineralisiertes Wasser dispergiert und weitere 10 min gerührt.

Die erhaltene Sprühsuspension wird wie in Beispiel 4 beschrieben auf Methylenblautabletten aufgetragen. Der Gesamtpolymerauftrag ist 4 mg/cm². Die Sprühzeit beträgt 110 min. Anschließend wird im Trockenschrank 40 °C über 4 Std. getrocknet. Die Tabletten zeigen einen glänzenden gleichmäßigen Überzug und zeigen im Zerfallstest nach Ph. Eur, in demineralisiertem Wasser und künstlichem Magensaft die in der Tabelle angegebenen Werte. Zusätzlich wurden die Zeiten für das Eindringen der Testflüssigkeit durch den Filmüberzug in den Tablettenkern durch Auftreten einer blauen Verfärbung festgestellt.

| | Polymerauftrag | | | |
|---|---|---|---|---|
| | 1 mg /cm² | 2mg/cm² | 4mg/cm² | unüberzogen |
| Zerfall in dem. Wasser | 0,3-1,5 min | 15->20 min | > 20 min | < 1 min |
| Zerfall in 0,1 N HCL | 16-18 sec | 20-32 sec | 40-52 sec | 10-37 sec |
| Zerfall in Darmsaft, pH 6,8 | 40 sec | 1-2 min | 2,5-7,5 min | < 1 min |
| Eindringen von Wasser | n.b. | >10min | > 20 min | sofort |

### 18. Isolierüberzug mit nicht erfindungsgemäßer Teilchengröße

Der in Beispiel 4 beschriebene Versuch wird mit gröberem Copolymer aus Methylmethacrylat, Butylmethacrylat, und Dimethylaminoethylmethacrylat in Verhältnis 25 : 25 : 50 (mittlere Teilchengröße 42 µm) wiederholt. Der Sprühauftrag verlief irregulär wegen starker Staubbildung. Der Überzug war nicht gleichmäßig und hatte eine rauhe, unregelmäßige Oberfläche.

### 19. Isolierüberzug ohne Weichmacherzusatz

Der in Beispiel 4 beschriebene Versuch wird ohne Zusatz von TEC wiederholt. Der Sprühauftrag verlief irregulär wegen starker Staubbildung. Der Überzug war nicht gleichmäßig und hatte eine rauhe, unregelmäßige Oberfläche.

### 20. Isolierüberzug ohne Tensidzusatz

Der in Beispiel 4. beschriebene Versuch wird ohne Zusatz von Polysorbat 80 Lösung wiederholt. Der Sprühauftrag verlief irregulär wegen starker Staubbildung. Der Überzug war nicht gleichmäßig und hatte eine rauhe, unregelmäßige Oberfläche.

## Patentansprüche

1. Verfahren zur Herstellung eines Überzugs- und Bindemittels für orale oder dermale Arzneiformen bestehend aus
(a) 35 - 98 Gew.-% eines Copolymers, bestehend aus radikalisch polymerisierten C1- bis C4-Estern der Acryl- oder Methacrylsäure und weiteren (Meth)acrylat-Monomeren die funktionelle tertiäre Ammoniumgruppen aufweisen und
(b) 1 - 50 Gew.-% eines Weichmachers sowie
(c) 1 - 15 Gew.-%, eines Emulgators mit einem HLB-Wert von mindestens 14
wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt werden und das Überzugs- und Bindemittel durch Schmelzen, Gießen, Ausstreichen oder Aufsprühen hergestellt wird,
**dadurch gekennzeichnet, daß**
das Copolymer (a) in Pulverform mit einer mittleren Teilchengröße von 1 - 40 µm eingebracht wird.

2. Verfahren nach Anspruch 1, **dadurch gekennzeichnet, daß** ein Trennmittel auf das Überzugs- und Bindemittel als Endschicht in konzentrierter Form aufgebracht wird

3. Verfahren nach Anspruch 1, **dadurch gekennzeichnet, daß** ein Pigment auf das Überzugs- und Bindemittel als Endschicht in konzentrierter Form aufgebracht wird.

4. Überzugs- und Bindemittel herstellbar nach dem Verfahren gemäß Anspruch 1.

5. Copolymer, bestehend aus radikalisch polymerisierten C1- bis C4-Estern der Acryl- oder Methacrylsäure und weiteren (Meth)acrylat-Monomeren die funktionelle tertiäre Ammoniumgruppen aufweisen, **dadurch gekennzeichnet, daß** es in Pulverform mit einer mittleren Teilchengröße von 1 - 40 µm vorliegt.

6. Copolymer nach Anspruch 5, **dadurch gekennzeichnet, daß** es aus 20 - 30 Gew.-% Methylmethacrylat, 20 - 30 Gew.-% Butylmethacrylat und 60 - 40 Gew.-% Dimethylaminoethylmethacrylat besteht.

7. Verwendung des Überzugs- und Bindemittels nach Anspruch 4 als geschmacksisolierender Überzug für wirkstoffhaltige pharmazeutische Zusammensetzungen

8. Verwendung des Überzugs- und Bindemittels nach Anspruch 4 als feuchtigkeitsisolierender Überzug für wirkstoffhaltige pharmazeutische Zusammensetzungen

9. Verwendung des Überzugs- und Bindemittels nach Anspruch 4 in einem transdermalen Therapiesystem.

## Claims

1. Process for preparing a coating and binding agent for oral or dermal pharmaceutical forms, consisting of
(a) 35 - 98 wt.% of a copolymer consisting of radically polymerised C₁₋₄-esters of acrylic or methacrylic acid and other (meth)acrylate monomers which comprise functional tertiary ammonium groups and
(b) 1 - 50 wt.% of a plasticiser and
(c) 1 - 15 wt.% of an emulsifier with an HLB value of at least 14
wherein components (a), (b) and (c) are mixed together with or without the addition of water and optionally with the addition of a pharmaceutically active substance and other conventional additives, and the coating and binding agent is produced by melting, casting, spreading or spraying,
**characterised in that**
the copolymer (a) is introduced in the form of a powder with an average particle size of 1 - 40 µm.

2. Process according to claim 1, **characterised in that** a release agent is applied in concentrated form to the coating and binding agent as the final layer.

3. Process according to claim 1, **characterised in that** a pigment is applied in concentrated form to the coating and binding agent as the final layer.

4. Coating and binding agent which may be prepared by the process according to claim 1.

5. Copolymer consisting of radically polymerised C₁₋₄-esters of acrylic or methacrylic acid and other (meth)acrylate monomers which comprise functional tertiary ammonium groups, **characterised in that** it is present in the form of a powder with an average particle size of 1 to 40 µm.

6. Copolymer according to claim 5, **characterised in that** it consists of 20 - 30 wt.% of methyl methacrylate, 20 - 30 wt.% of butyl methacrylate and 60 - 40 wt.% of dimethylaminoethyl methacrylate.

7. Use of the coating and binding agent according to claim 4 as a flavour-insulating coating for pharmaceutical compositions containing an active substance.

8. Use of the coating and binding agent according to claim 4 as a moisture-insulating coating for pharmaceutical compositions containing an active substance.

9. Use of the coating and binding agent according to claim 4 in a transdermal therapy system.

## Revendications

1. Procédé de production d'un enrobant et excipient pour formes médicamenteuses orales ou cutanées constitué de
A. 35 à 98 % en poids d'un copolymère constitué d'esters en C₁ à C₄, polymérisés par voie radicalaire, de l'acide acrylique ou de l'acide méthacrylique et d'autres monomères de (méth)acrylate qui présentent des groupes ammonium tertiaires fonctionnels et
B. 1 à 50 % en poids d'un plastifiant ainsi que
C. 1 à 15 % en poids d'un émulsifiant ayant une valeur d'EHL d'au moins 14,
les composants (a), (b) et (c) étant mélangés entre eux avec ou sans addition d'eau et le cas échéant avec addition d'une substance active pharmaceutique et d'autres additifs habituels, et l'enrobant et excipient étant produit par fusion, coulée, étalement ou pulvérisation,
**caractérisé en ce que**
le copolymère (a) est introduit sous forme de poudre avec une taille particulaire moyenne de 1 à 40 µm.

2. Procédé selon la revendication 1,
**caractérisé en ce qu'**
on dépose un agent de séparation sur l'enrobant et excipient comme couche finale sous forme concentrée.

3. Procédé selon la revendication 1,
**caractérisé en ce qu'**
on dépose un pigment sur l'enrobant et excipient comme couche finale sous forme concentrée.

4. Enrobant et excipient pouvant être produit selon la procédé de la revendication 1.

5. Copolymère constitué d'esters en C₁ à C₄ , polymérisés par voie radicalaire, de l'acide acrylique ou de l'acide méthacrylique ou d'autres monomères de (méth)acrylate qui présentent des groupes ammonium tertiaires fonctionnels,
**caractérisé en ce qu'**
il se présente sous forme de poudre avec une taille particulaire moyenne de 1 à 40 µm.

6. Copolymère selon la revendication 5,
**caractérisé en ce qu'**
il se compose de 20 à 30 % en poids de méthacrylate de méthyle, de 20 à 30 % en poids de méthacrylate de butyle et de 60 à 40 % en poids de méthacrylate de diméthylaminoéthyle.

7. Utilisation de l'enrobant et excipient selon la revendication 4, comme revêtement gustativement isolant pour compositions pharmaceutiques contenant une substance active.

8. Utilisation de l'enrobant et excipient selon la revendication 4, comme revêtement isolant vis-â-vis de l'humidité pour compositions pharmaceutiques contenant une substance active.

9. Utilisation de l'enrobant et excipient selon la revendication 4, dans un système thérapeutique transdermique.
